# EUROPEAN PATENT APPLICATION

(11) **EP 2 671 945 A1**
(43) Date of publication of application: **11.12.2013**
(21) Application number: 11857898.8
(22) Date of filing: 24.08.2011
(51) Int. Cl.: C12N 5/10, C12N 5/074

(54) **METHOD FOR CULTURING HUMAN PLURIPOTENT STEM CELLS**

(30) Priority: 31.01.2011 JP 2011019109; 23.05.2011 JP 2011115150
(71) Applicant: National Institute of Biomedical Innovation, Ibaraki-shi, Osaka 567-0085 (JP)
(72) Inventor: FURUE, Miho, Ibaraki-shi Osaka 567-0085 (JP); KINEHARA, Masaki, Ibaraki-shi Osaka 567-0085 (JP)
(74) Representative: Harding, Charles Thomas
(86) International application number: PCT/JP2011/004691
(87) International publication number: WO 2012/104936

(57) **Abstract**

An aim of the present invention is to provide a method for culturing a human pluripotent stem cell while maintaining an undifferentiated state, more efficiently than conventional methods, and a kit therefor. The pluripotency of a stem cell was found to be maintained at a high rate, regardless of experimenter's proficiency in culturing techniques, by (a) culturing a human pluripotent stem cell in a first medium which is a medium for pluripotent stem cell comprising activin; (b) replacing the first medium with a second medium which is a medium for pluripotent stem cell comprising no activin, and culturing the human pluripotent stem cell, (c) subculturing the human pluripotent stem cell into the first medium, and then repeating the above (b) and (c) sequentially.

## Description

### Technical Field

The present invention relates to a method for culturing a human pluripotent stem cell while maintaining an undifferentiated state and a kit therefor, and more particularly, the present invention relates to a method for culturing a human pluripotent stem cell while maintaining an undifferentiated state and a kit therefor, the method comprising: a step (a) of culturing a human pluripotent stem cell in a first medium comprising a basal medium for pluripotent stem cell supplemented with an activin-containing supplement; a step (b) of replacing the first medium with a second medium comprising the basal medium for pluripotent stem cell supplemented with an activin-free supplement and culturing the human pluripotent stem cell; and a step (c) of subculturing the human pluripotent stem cell into the first medium; wherein after the step (a), the steps (b) and (c) are repeated in the order of (a), (b), (c), (b), (c), and so on.

### Background Art

Pluripotent stem cells such as ES cells are capable of differentiating into various tissues, and therefore considered to be applied to models for elucidating the tissue differentiation process, regenerative medicine, and the like. In order to precisely analyze the functions and effects of differentiation-inducing factors, controlling the cellular differentiation is important.

Proposed methods for maintaining an undifferentiated state of stem cells include a method for culturing pluripotent stem cells, comprising a step of culturing pluripotent stem cells, while suppressing the differentiation of the pluripotent stem cells by subjecting the pluripotent stem cells to n-axis rotations (wherein n is an integer of 2 or more) (see for example, Patent Document 1); a medium for maintaining pluripotency of mesenchymal stem cells, comprising TGF-β (see for example, Patent Document 2); a serum-free medium comprising a basal medium which allows to conduct primary culture and subculture of stem cells without serum, mixed with pannexin at a concentration of 5 to 10%, 1 to 100 ng/mL bFGF, 1 to 100 ng/mL PDGF, 1 to 100 ng/mL EGF, and 1 to 1000 µg/mL vitamin C (see for example, Patent Document 3); a method for maintaining embryonic stem cells, comprising culturing embryonic stem cells in suspension in a serum-free medium containing polyvinyl alcohol (PVA) and being free of animal-derived albumin, in the absence of a feeder cell (see for example, Patent Document 4); a tissue stem cell growing agent containing a low-molecular weight compound or its salt as an active ingredient (see for example, Patent Document 5); a stem cell differentiation inhibitor comprising a low molecular weight compound such as indole derivatives as an active ingredient (see for example, Patent Document 6); and a medium for long-term growth and development of a cell, including an embryonic-stem cell, comprising, in effective amounts for culturing the cell, (a) a standard medium, (b) serum albumin, (c) transferrin, (d) a lipid and fatty acid source, (e) cholesterol, (f) a reducing agent, (g) a pyruvate salt, (h) nucleosides for DNA and RNA synthesis, (i) at least one growth factor that stimulates the growth and development of a substrate cell, a tissue cell or an organ cell, and (j) at least one extracellular matrix material, wherein the medium is serum-free or of low serum (see for example, Patent Document 7). A method for culturing pluripotent stem cells comprising culturing the pluripotent stem cells in the presence of a decidua-derived cell or an extracellular matrix derived from the cell (see for example, Patent Document 8) has also been proposed. It is reported, however, that while cell growth on fibronectin was observed at a low degree, the degree was markedly lower than that of cell growth on the extracellular matrix of mesenchymal cells derived from the decidua.

The present inventors have developed a culture medium for serum-free medium that is capable of culturing ES cells in a type I collagen-coated flask for a prolonged period of time in the absence of feeder cells, while maintaining the ES cells in an undifferentiated state under serum-free conditions, and a basal medium for producing such a culture medium (see for example, Patent Document 9); and a method for maintaining a primate embryonic stem cells under cell culture conditions in the absence of feeder cells or serum, comprising: i) a step of preparing primate embryonic stem cells using a culture vessel coated with a basal culture support consisting of protein, wherein the cells are cultured in the culture vessel by a cell culture medium comprising: bovine insulin, human transferrin, sodium selenite, ethanolamine, 2-mercaptoethanol, oleic acid complexed with fatty acid-free bovine albumin and wherein the cell culture medium is further supplemented with fibroblast growth factor, heparin and ascorbic acid or ascorbic acid phosphate or derivative thereof; and ii) maintaining the primate embryonic stem cells in an undifferentiated state (see for example, Patent Document 10). Moreover, as to respective matrix ingredients, it was found, in the development of serum-free medium, that laminin and fibronectin act suppressive to a state maintaining the undifferentiation of murine ES cells, and act to promote the differentiation (see for example, Non-patent Document 1).

Moreover, using Shef human ES cells and the cell line HUES-1, the present inventors found that human ES cells can be maintained and cultured with a type I collagen coating, and that the addition of HEPES into a serum-free medium has a very adverse effect on the maintenance of human ES cells (see for example, Non-patent Document 2), further, the present inventors also reported that heparin, which is known to aid FGF-2 action of promoting the proliferation of ES cells, promotes the proliferation of ES cells even when added in the absence of FGF-2 (see for example, Non-patent Document 3); found that the addition of activin activates ERK (extracellular signal-regulated kinases) and promotes differentiation induction into the nerve or mesendoderm, while the addition of ERK inhibitors can maintain an undifferentiated state (see for example, Non-patent Document 4); and reviewed a basic method for culturing human ES cells (see for example, Non-patent Document 5).

### Prior Art Documents

### Patent Documents

Patent Document 1: Japanese unexamined Patent Application Publication No. 2010-193910
Patent Document 2: Japanese unexamined Patent Application Publication No. 2010-094062
Patent Document 3: Japanese unexamined Patent Application Publication No. 2008-148643
Patent Document 4: Japanese unexamined Patent Application Publication No. 2007-228815
Patent Document 5: Japanese unexamined Patent Application Publication No. 2006-180763
Patent Document 6: Japanese unexamined Patent Application Publication No. 2005-013152
Patent Document 7: Japanese unexamined Patent Application Publication (Translation of PCT Application) No. 8-508891
Patent Document 8: Japanese unexamined Patent Application Publication No. 2010-166901
Patent Document 9: International Publication No. WO2005/063968
Patent Document 10: Japanese unexamined Patent Application Publication (Translation of PCT Application) No. 2009-542247

### Non-patent Documents

Non-patent Document 1: O-07 Functional analysis of matrix ingredients in differentiation control of murine ES cells, The 52nd Annual Meeting of the Japanese Matrix Club (2005) Oita
Non-patent Document 2: PNAS, vol.105, no.36, 13409-13414
Non-patent Document 3: In Vitro Cell.Dev. Biol.- Animal (2010) 46:573-576
Non-patent Document 4: Stem Cell Res. 2010
Non-patent Document 5: Tiss. Cult. Res Commun.27;139-147 (2008)

### Summary of the Invention

### Object to be Solved by the Invention

An aim of the present invention is to provide a method for culturing a human pluripotent stem cell while maintaining an undifferentiated state, more efficiently than conventional methods, and a kit therefor.

### Means to Solve the Object

As described in the foregoing, the development of serum-free medium that allows to culture pluripotent stem cells such as ES cells for a long period of time has been progressed in many aspects. Growing pluripotent stem cells in these media requires high level of culturing technique, though it is not impossible, often depending on intuition and know-how of experimenters, to avoid frequent cell death or loss of pluripotency in the course of culturing.

Conventional media usually comprise animal-derived materials. For example, use of bovine serum albumin, porcine-derived type I collagen, and the like has a problem of being high cost with limited supply. Also, the animal-derived materials are usually unheated blood products, and possible to be contaminated with prion, which is considered to be the cause of bovine spongiform encephalopathy (BSE), pathogens such as viruses, and heterogeneous ingredients, raising safety and immunogenicity issues. Furthermore, lot to lot variations in the production of such a medium is considered to be one of the causes that lower the probability of culturing while maintaining an undifferentiated state.

Based on these findings, the present inventors have tried to prepare a medium that allows to culture pluripotent stem cells with high safety and no lot to lot variation while maintaining an undifferentiated state by substituting or deleting animal-derived ingredients. They have carefully examined ingredients one by one, since it was known that, for example, in the case of substitution of an animal-derived ingredient with a recombinant generated by genetic modification technologies, a recombinant ingredient may have a different structure from the conventional ingredient derived from an animal or may show an immunogenic behavior different from the conventional material derived from an animal. Since it was found that an undifferentiated state can be maintained by adding an ERK inhibitor into a medium comprising activin, as described above, the present inventors continued detailed examinations on the relation between activin and other ingredients, and surprisingly found that the pluripotency of a stem cell is maintained at a high rate, regardless of experimenter's proficiency in culturing techniques, by (a) culturing a human pluripotent stem cell in a first medium comprising a basal medium for pluripotent stem cell supplemented with an activin-containing supplement, (b) replacing the first medium with a second medium comprising the basal medium for pluripotent stem cell and an activin-free supplement, and culturing the human pluripotent stem cell, and (c) subculturing the human pluripotent stem cell into the first medium, and then repeating the above (b) and (c) sequentially. It was found that use of such a method allows to subculture pluripotent stem cells for a prolonged period of time, while maintaining an undifferentiated state of the cells, with a proportion of more than 4 to 5 out of 5 to 6 people in a laboratory, in contrast to conventionally known methods which allow 1 out of 5 to 6 people in a laboratory to maintain the culture of pluripotent stem cells. The present inventors continued the examination on ingredients that increase the proportion of pluripotent stem cells maintained in an undifferentiated state, and found that the addition of a protein kinase C inhibitor into the medium increased probability of cells being maintained in an undifferentiated state, and eliminated the necessity of mechanically removing a portion of differentiated cells. The method for culturing human pluripotent stem cells according to the present invention has thus been completed based on the foregoing findings.

Accordingly, the present invention relates to: [1] a method for culturing a human pluripotent stem cell while maintaining an undifferentiated state, comprising: (a) a step of culturing a human pluripotent stem cell in a first medium comprising a basal medium for pluripotent stem cell supplemented with an activin-containing supplement; (b) a step of replacing the first medium with a second medium comprising the basal medium for pluripotent stem cell supplemented with an activin-free supplement, and culturing the human pluripotent stem cell; and (c) a step of subculturing the human pluripotent stem cell into the first medium, wherein after the step (a), the steps (b) and (C) are sequentially repeated; [2] the method according to [1], wherein the supplements comprise fibronectin, insulin, transferrin, 2-mercaptoethanol, 2-ethanolamine, sodium selenite and an albumin-conjugated oleic acid; [3] the method according to [2], wherein fibronectin is used without being coated on an inside of a culture vessel; [4] the method according to [2] or [3], wherein the supplements further comprise a protein kinase C inhibitor; [5] the method for culturing according to any one of [1] to [4], wherein the basal medium is a chemically defined medium comprising one or more sugars, one or more inorganic salts, one or more amino acids, one or more vitamins and a trace ingredient; and [6] the method for culturing according to any one of [1] to [4], wherein the basal medium is a hESF-grow medium shown in Table 1:

**[Table 1]**

| hESF-grow medium | | | |
|---|---|---|---|
| INORGANIC SALTS | mg/L | VITAMINS | mg/L |
| CaCl₂ | 108.305 | Ascorbic acid 2-phosphate | 100 |
| Ca (NO₃)₂ | 25 | D-Biotin | 0.05185 |
| CuSO₄·5H₂O | 0.000625 | Choline Chloride | 6.24 |
| Fe (NO₃)₃·9H₂O | 0.05 | Folic Acid | 2.575 |
| FeSO₄·7H₂O | 0.2085 | I-Inositol | 16.85 |
| MgCl·6H₂O | 30.515 | Niacinamide | 2.25925 |
| MgSO₄ | 61.055 | D-Pantothenic Acid·½Ca | 2.1825 |
| KCl | 355.9 9 | Pyridoxal·HCl | 2 |
| NaCl | 6599.75 | Pyridoxine·HCl | 0.2655 |
| Na₂HPO₄ 2H₂O | 295.28 | Riboflavin | 0.2595 |
| NaH₂PO₄ H₂O | 61.51 | Thiamine·HCl | 2.335 |
| ZnSO₄·7H₂O | 0.216 | Vitamin B-12 | 0.34125 |
| AMINO ACIDS | mg/L | p-aminobenzoic acid | 0.25 |
| L-Alanine | 2.225 | | |
| L-Arginine | 50 | MISC. | mg/L |
| L-Arginine·HCl | 94.75 | NaHCO₃ | 2000 |
| L-Asparagine·H₂O | 16.2525 | Gluthathione | 0.25 |
| L-Aspartic Acid | 8.325 | Thymidine | 0.1825 |
| L-Cystine·HCl·H₂O | 47.5725 | Hypoxanthine | 1.02 |
| L-Cysteine·2HCl | 7.88 | Lipoic acid | 0.0525 |
| L-Glutamic Acid | 8.675 | Linoleic acid | 0.021 |
| L-Glutamine | 549.65 | Phenol Red·Na | 6.56 |
| Glycine | 19.375 | Putrescine·2HCl | 0.04025 |
| L-Histidine·HCl· | 23.165 | Pyruvic acid·Na | 110 |
| L-Hydroxyproline | 5 | | |
| L-Isoleucine | 65.935 | D-Glucose | 2000 |
| L-Leucine | 68.225 | | |
| L-Lysine·HCl | 92.175 | | |
| L-Methionine | 19.87 | | |
| L-Phenylalanine | 37.99 | | |
| L-Proline | 13.625 | | |
| L-Serine | 31.125 | | |
| L-Threonine | 55.525 | | |
| L-Tryptophan | 9.76 | | |
| L-Tyrosine· | 42.36 | | |
| L-Valine | 54.825 | | |

Furthermore, the present invention relates to: [7] a kit for culturing a human pluripotent stem cell while maintaining an undifferentiated state, comprising an activin-containing supplement, an activin-free supplement and an ingredient of basal medium for human pluripotent stem cell; [8] the kit according to [7], wherein the supplements comprise fibronectin, insulin, transferrin, 2-mercaptoethanol, 2-ethanolamine, sodium selenite and an albumin-conjugated oleic acid oleic acid; [9] the kit according to [7] or [8], wherein fibronectin is added to the supplements; [10] the kit according to any one of [7] to [9], wherein the supplements further comprise a protein kinase C inhibitor; [11] the kit according to any one of [7] to [10], wherein the basal medium is a chemically defined medium comprising one or more sugars, one or more inorganic salts, one or more amino acids, one or more vitamins and a trace ingredient; [12] the kit according to any one of [7] to [10], wherein the basal medium is the hESF-grow medium shown in Table 1; and [13] the kit according to any one of [7] to [12], further comprising an antibody, a probe or a primer against an undifferentiation and/or differentiation marker.

### Effect of the Invention

By using the method according to the present invention, human pluripotent stem cells can be cultured and grown while maintaining an undifferentiated state. Such pluripotent stem cells maintained in an undifferentiated state can be induced to differentiate into cells of interest by treating under suitable conditions.

### Brief Description of Drawings

[Figure 1] Figure 1 shows results of measuring the adhesion ability of respective cells to collagen I, gelatin, laminin, and fibronectin. Each of the results of human ES cell line KhES-1 (a), human iPS cell line Tic (JCRB1331) (b) and embryonal cancer cell line PA-1 (JCRB9061) (c) are shown.
[Figure 2] Figure 2 shows graphs of the analysis on percentages of positive TRA-1-60, SSEA-1, SSEA-4, or Nanog expression cells by fluorescent immunostaining of cells obtained by culturing ES cell line H9 (WA09) in a medium comprising hESF9 medium supplemented with oleic acid and/or BSA or rHSA. The values when cultured under culture condition (1) are expressed as "1" and ratios to the values are shown for the rest.

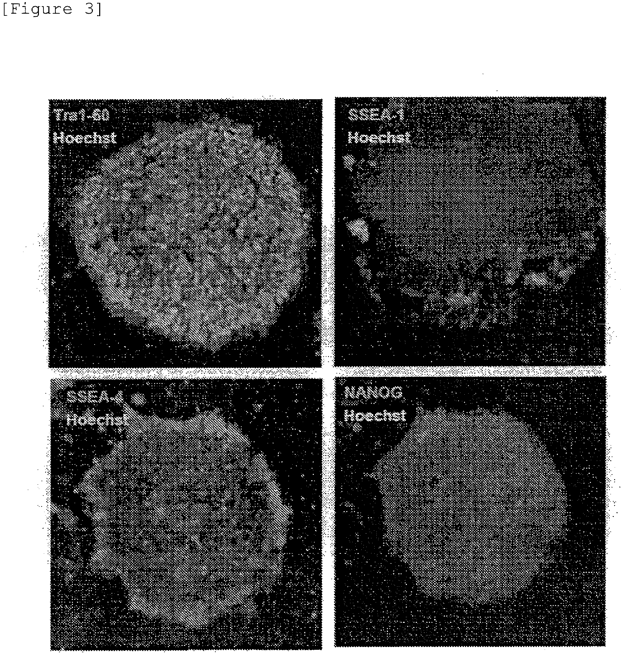

[Figure 3] Figure 3 shows micrographs of cells obtained by culturing ES cell line H9 (WA09) in a medium supplemented with oleic acid conjugated with rHSA, and fluorescent immunostaining of the cells with TRA-1-60, SSEA-1, SSEA-4, or Nanog.
[Figure 4] Figure 4 shows the state of cells of the cell line H9 (WA09) at passage 2 cultured in a medium in which heparin is removed from hESF-9.
[Figure 5] Figure 5 shows micrographs of the ES cell line H9 (WA09) cultured in an activin-containing medium (a) and after medium change into an activin-free medium (b).
[Figure 6] Figure 6 shows micrographs of cells of the ES cell line H9 (WA09) at passage 1 to 6 cultured by the method according to the present invention.
[Figure 7] Figure 7 shows expression of undifferentiation and differentiation markers in the ES cell line H9 (WA09) at passage 5 cultured by the method according to the present invention. SSEA-3, SSEA-4, TRA-2-54, TRA-1-60, TRA-1-80, and CD90 indicate expression of an undifferentiation marker, and SSEA-1, CD105, CD56, and A2B5 indicate expression of a differentiation marker.
[Figure 8] Figure 8 shows the state of cells cultured in a medium supplemented with 0, 20, 40, 80, 160, or 320 µg/well of fibronectin by the method according to the present invention.
[Figure 9] Figure 9 shows the state of cells cultured in a medium supplemented with 0, 1, 2.5, 5, or 10 µM of myristoylated protein kinase C peptide inhibitor.

### Mode of Carrying Out the Invention

A method for culturing a human pluripotent stem cell while maintaining an undifferentiated state according to the present invention is not particularly limited, as far as it is a method comprising: (a) a step of culturing a human pluripotent stem cell in a first medium comprising a basal medium for pluripotent stem cell supplemented with an activin-containing supplement; (b) a step of replacing the first medium with a second medium comprising the basal medium for pluripotent stem cell supplemented with an activin-free supplement, and culturing the human pluripotent stem cell; and (c) a step of subculturing the human pluripotent stem cell into the first medium; wherein after the step (a), the steps (b) and (C) are sequentially repeated. Also, a kit for culturing a human pluripotent stem cell while maintaining an undifferentiated state according to the present invention is not particularly limited, as far as it is a kit comprising a supplement and a basal medium ingredient for pluripotent stem cell. "Medium" herein refers to "medium ingredients" that allow to culture cells, in a state where water is added thereto. "While maintaining an undifferentiated state" herein refers to a state maintaining the properties of pluripotent cells to maintain self-renewal capacity, and to have ability to differentiate into all types of cells present in the living body.

The present invention is directed to human pluripotent stem cells. Examples of the pluripotent stem cells include embryonic stem cells (ES cells), which are isolated from early embryos; embryonic germ cells (EG cells), which are isolated from primordial germ cells at a fetal stage (see for example, Proc Natl Acad Sci USA. 1998, 95:13726-31); germline stem cells (GS cells), which are isolated from neonatal testes(see for example, Nature. 2008, 456:344-9); stem cells derived from bone marrows such as iliac bone marrow and jawbone marrow; mesenchymal stem cells such as stem cells derived from adipose tissue; and artificial pluripotent stem cells derived from somatic cells (or induced pluripotent stem cells (iPS cells)), which are obtained by inducing dedifferentiation of somatic cells from subjects themselves by introducing a plurality of genes into the somatic cells such as skin cells, and have pluripotency equal to that of ES cells; and particularly, human ES cells, such as human ES cell line H9 (WA09), human ES cell line H1 (WA01: National Stem Cell bank, WISC Bank) and KhES-1, KhES-2 and KhES-3 (all from Laboratory of embryonic stem Cell research, Institute for Frontier Medical Sciences, Kyoto University), HES3, HES4 and HES6 (National Stem Cell bank, Monash University); iPS cells such as iPS cells obtained by introducing Oct3/4, Klf4, C-Myc and Sox2 genes (Riken BioResource Center; Kyoto University), iPS cell line Tic (JCRB1331), iPS cell line Dotcom (JCRB1327), Squeaky (JCRB1329) and iPS cell line Toe (cell line JCRB1338), iPS cell line Lollipop (cell line JCRB1336) (from National Center for Child Health and Development; JCRB cell bank, Research on Disease Bioresources, National Institute of Biomedical Innovation), iPS cell lines UTA-1 and UTA-1-SF-2-2 (both from University of Tokyo), iPS cells obtained by introducing Oct3/4, Klf4 and Sox2 genes (Nat Biotechnol 2008; 26: 101-106).

The basal medium for pluripotent stem cell used in the present invention is not particularly limited, as far as it is a medium that allows to culture human pluripotent stem cells while maintaining an undifferentiated state when supplemented with the supplement according to the present invention, but preferably it is easy to prepare, preferably a chemically defined medium for preventing lot to lot variation, preferably includes one or more sugars, one or more inorganic salts, one or more amino acids, one or more vitamins and one or more trace ingredients, and may include antibiotics such as kanamycin, as appropriate, to be used for drug susceptibility test.

Examples of the aforementioned sugars specifically include monosaccharides such as glucose, lactose, mannose, fructose, and galactose, and disaccharides such as sucrose, maltose and lactose, and combinations of one or more of these sugars are also available, but glucose is particularly preferable among these.

Examples of the aforementioned inorganic salts specifically include one or more inorganic salts selected from calcium chloride, calcium nitrate, copper sulfate pentahydrate, iron nitrate (III) nonahydrate, ferrous sulfate (II) heptahydrate, magnesium chloride hexahydrate, magnesium sulfate, potassium chloride, sodium chloride, disodium hydrogenphosphate, disodium hydrogenphosphate dihydrate, sodium dihydrogen phosphate, sodium dihydrogen phosphate dihydrate, and zinc sulfate heptahydrate. Any of inorganic salts or combinations thereof can be used, as far as they are ingredients that act favorably for maintaining an undifferentiated state of pluripotent stem cells.

Examples of the aforementioned amino acids specifically include one or more amino acids selected from alanine, arginine, asparagine, aspartic acid, cystine, cysteine, glutamine, glycine, histidine, glutamic acid, hydroxyproline, isoleucine, leucine, lysine, methionine, phenylalanine, proline, serine, threonine, tryptophan, tyrosine, and valine. Preferably, the amino acids are L-amino acids, derivatives thereof and salts thereof, and their derived compounds, such as hydrates thereof. Examples of the aforementioned arginine include, for example, arginine-derived compounds, such as L-arginine hydrochloride and L-arginine monohydrochloride. Examples of the aforementioned aspartic acid include aspartic acid-derived compounds, such as sodium L-aspartate salt monohydrate, L-aspartic acid monohydrate, potassium L-aspartate, and magnesium L-aspartate. Examples of the aforementioned cysteine include cysteine-derived compounds, such as L-cysteine dihydrochloride, and L-cysteine hydrochloride monohydrate, and lysine-derived compounds, such as L-lysine hydrochloride. Examples of the aforementioned glutamic acid include glutamine-derived compounds, such as L-glutamic acid monosodium salt. Examples of the aforementioned asparagine include asparagine-derived compounds, such as L-asparagine monohydrate. Examples of the aforementioned tyrosine include tyrosine-derived compounds, such as L-tyrosine disodium dihydrate. Examples of the aforementioned histidine include histidine-derived compounds, such as histidine hydrochloride and histidine hydrochloride monohydrate. Examples of the aforementioned lysine include lysine-derived compounds such as L-lysine hydrochloride.

Examples of the aforementioned vitamins specifically include one or more vitamins selected from ascorbic acid, biotin, choline, folic acid, inositol, niacin, pantothenic acid, pyridoxine, riboflavin, thiamine, vitamin B12, and para-aminobenzoic acid (PABA), derivatives of these ingredients and salts thereof, and their derived compounds such as hydrates thereof. Examples of the aforementioned ascorbic acid include, for example, ascorbic acid-derived compounds such as ascorbic acid 2-phosphate, magnesium ascorbyl phosphate, sodium ascorbyl sulfate, aminopropyl ascorbyl phosphate, and sodium ascorbyl phosphate. Examples of the choline include choline-derived compounds, such as choline chloride. Examples of the niacin include niacin-derived compounds, such as nicotinic acid, nicotinic acid amide and nicotinic alcohol. Examples of the pantothenic acid include pantothenic acid-derived compounds, such as calcium pantothenate, sodium pantothenate, and panthenol. Examples of the pyridoxine include pyridoxine-derived compounds, such as pyridoxine hydrochloride, pyridoxal hydrochloride, phosphate pyridoxal, and pyridoxamine. Examples of the thiamine include thiamine-derived compounds, such as thiamine hydrochloride, thiamine nitrate, bisthiamine nitrate, thiamine dicetyl sulfate ester salt, fursultiamine hydrochloride, octotiamine, and benfotiamine. The aforementioned ascorbic acid is preferred to be added.

Preferably, the aforementioned trace ingredients are ingredients that act favorably for maintaining an undifferentiated state of pluripotent stem cells, including ingredients generally used as a medium ingredient such as glutathione, hypoxanthine, lipoic acid, linolenic acid, phenol red, putrescine, pyruvic acid, thymidine, and NaHCO₃, and derivatives of these ingredients and salts thereof, and their derived compounds such as hydrates thereof, for example, putrescine dihydrochloride, and sodium pyruvate.

Specific examples of the aforementioned basal medium for human pluripotent stem cell include media such as publicly-known chemically defined media such as commercial Dulbecco's modified Eagle medium (DMEM), minimum essential medium (MEM), basal medium of Eagle (BME), RPMI1640 medium, F12 medium; media in which any two or more of these media are mixed in a suitable ratio, such as DMEM/F12 medium (the medium in which DMEM and F12 media are mixed at 1:1); and media in which these media are supplemented with the aforementioned ascorbic acid, preferably ascorbic acid 2-phosphate. Preferable examples particularly include basal media of the compositions shown in Table 1 above (hereafter, also referred to as "hESF-grow medium") as an animal product-free basal medium.

Moreover, examples of the concentrations (contents) of the individual medium ingredients above, for example, those of the individual ingredients of hESF-grow medium, include the concentration range of 0 to 200 for each ingredient, preferably the concentration range of 40 to 160, more preferably the concentration range of 80 to 120, and further more preferably the concentration range of 90 to 110, as compared to the concentrations of respective ingredients shown in the composition of hESF-grow medium above, which is taken as 100. For example, for L-arginine, preferable examples of its concentration include a concentration of 0 to 100 mg/L, preferably a concentration of 20 to 80 mg/L, more preferably a concentration of 40 to 60 mg/L, and further more preferably a concentration of 45 to 55 mg/L.

The supplements for use in the method according to the present invention are not particularly limited, as far as the addition of such a supplement to the aforementioned basal medium for pluripotent stem cell produces a medium that allows to culture human pluripotent stem cells while maintaining an undifferentiated state. Specific examples include those comprising an ingredient such as fibronectin, insulin, transferrin, 2-mercaptoethanol, 2-ethanolamine, selenium, FGF-2, albumin-conjugated oleic acid, a protein kinase C inhibitor. The supplements may be used as a combination of multiple separate supplements, each consisting of one or more of the aforementioned ingredients, or as a single combined supplement comprising many of the aforementioned ingredients.

The first medium for use in the method according to the present invention is not particularly limited, as far as the addition of the activin-containing supplement to the aforementioned basal medium for pluripotent stem cell produces a medium that allows to culture human pluripotent stem cells. The second medium for use in the method according to the present invention is not particularly limited, as far as the addition of the activin-free supplement to the aforementioned basal medium for pluripotent stem cell produces a medium that allows to culture human pluripotent stem cells. The aforementioned activin-containing supplement is not particularly limited, as far as it is a supplement comprising activin, but preferably a supplement containing fibronectin. Examples of the aforementioned supplements include supplements comprising one or more ingredients selected from fibronectin, insulin, transferrin, 2-mercaptoethanol, 2-ethanolamine, selenium, FGF-2, albumin, oleic acid, and a protein kinase C inhibitor; and supplements comprising a combination of fibronectin and one or more ingredients selected from insulin, transferrin, 2-mercaptoethanol, 2-ethanolamine, selenium, FGF-2, albumin, oleic acid, and a protein kinase C inhibitor. Preferable examples include, for example, a supplement comprising fibronectin, insulin, transferrin, 2-mercaptoethanol, 2-ethanolamine, selenium, FGF-2, albumin and the oleic acid; and a supplement comprising these and further comprising a protein kinase C inhibitor.

In the aforementioned first medium, fibronectin may be applied to the inside of culture vessels as a coating and used as an ingredient constituting a supplement to be added to the aforementioned basal medium for pluripotent stem cell to increase the cell-culture vessel adhesiveness, or may be added together with other supplement ingredients to the basal medium without coating the inside of the culture vessel. Specifically, a first medium (hereafter also referred to as, "first medium I") can be prepared by coating a culture vessel with fibronectin, and then adding activin and the aforementioned supplement without fibronectin to a basal medium; but a preferable first medium I is prepared by coating a culture vessel with a fibronectin, and then adding a combination of activin and a mixture of insulin, transferrin, 2-mercaptoethanol, 2-ethanolamine, selenium, FGF-2, albumin and oleic acid to a basal medium, and more preferably further adding a protein kinase C inhibitor. When using fibronectin without coating the inside of the culture vessel, a first medium (hereafter also referred to as, "first medium II") can also be prepared by adding activin and the aforementioned supplement to a basal medium together.

A second medium (hereafter also referred to as, "second medium I") can be prepared by coating the inside of a culture vessel with fibronectin, and then adding the aforementioned supplement without fibronectin to a basal medium; but a preferable second medium I is prepared by coating a culture vessel with fibronectin, and then adding insulin, transferrin, 2-mercaptoethanol, 2-ethanolamine, selenium, FGF-2, albumin and oleic acid, and more preferably further adding a protein kinase C inhibitor. A second medium (hereafter also referred to as, "second medium II") can also be prepared by adding the aforementioned supplement to a basal medium; but a preferable second medium II is prepared by adding fibronectin, insulin, transferrin, 2-mercaptoethanol, 2-ethanolamine, selenium, FGF-2, albumin and oleic acid to a basal medium together, and more preferably further adding a protein kinase C inhibitor. When the aforementioned first medium I is to be used, then using the aforementioned second medium I is preferable, and when the aforementioned first medium II is to be used, then using the aforementioned second medium II is preferable.

The respective ingredients constituting the aforementioned activin-containing supplement and/or the activin-free supplement are preferably of animal product-free grade. Examples of the ingredients of animal product-free grade include recombinant proteins artificially produced and purified by genetically-modified technologies, chemically synthesized products, and plant-derived ingredients that are not animal-derived ingredients, but act similarly to animal-derived ingredients. Examples of the aforementioned recombinant proteins also include proteins whose structures are not same as those of animal-derived ingredients, such as tagged proteins. Examples of the chemically synthesized products include derivatives thereof and salts thereof, and hydrates thereof.

Examples of the aforementioned fibronectin include naturally derived fibronectin such as porcine-derived fibronectin, bovine-derived fibronectin, or human-derived fibronectin, known as a type of extracellular matrix (ECM); and fibronectin of animal product-free grade, including recombinant fibronectin, such as a form of fibronectin made from recombinant DNA that is identical or modified to bovine, porcine or human fibronectin.

When the aforementioned fibronectin is used as a coating, it can be used with well-known methods, such as physical adsorption; and introduction of a reactive functional group into the cell adhesion region of the culture vessel and immobilization of a cell adhesion factor onto the surface of the vessel (the inside of the vessel) by chemical bond. Specifically, examples include a method of applying a solution comprising fibronectin to a culture vessel, for example, at a concentration of 0.5 to 5 µg/cm², preferably 1 to 3 µg/cm², more preferably, 1.5 to 2.5 µg/cm²; allowing to stand with avoiding being dried, for example at 37°C, for 1 to 12 hours, preferably for 2 to 8 hours, and more preferably for 3 to 5 hours; and aspirating the aforementioned solution just before inoculating pluripotent stem cells. The first medium and/or the second medium according to the present invention can be prepared by adding the ingredients other than fibronectin of the activin-containing supplement or the activin-free supplement to the aforementioned basal medium for pluripotent stem cell, and preparing the medium on the fibronectin coating.

When using the aforementioned fibronectin as a medium ingredient, the first medium and/or the second medium for use in the present invention can be prepared by adding the aforementioned fibronectin as an ingredient constituting the aforementioned activin-containing supplement and/or the activin-free supplement to the aforementioned basal medium for human pluripotent stem cell, for example, at a concentration of 1.0 to 100 µg/mL, preferably 5 to 75 µg/mL, preferably 10 to 50 µg/mL, and more preferably 25 to 45 µg/mL.

Examples of the aforementioned insulin include naturally derived insulin such as porcine-derived insulin, bovine-derived insulin, or human-derived insulin; and insulin of animal product-free grade, including recombinant insulin, such as a form of insulin made from recombinant DNA that is identical or modified to bovine, porcine or human insulin. Preferable examples particularly include human recombinant insulin (recombinant human insulin). The first medium and/or the second medium for use in the present invention can be prepared by adding the insulin as an ingredient constituting the aforementioned activin-containing supplement and/or the activin-free supplement to the aforementioned basal medium for human pluripotent stem cell, at a final concentration of 1 to 20 µg/mL, preferably 5 to 15 µg/mL, more preferably 7.5 to 12.5 µg/mL, and more preferably 9 to 11 µg/mL.

Example of the aforementioned transferrin include naturally derived transferrin, such as porcine-derived transferrin, bovine-derived transferrin, or human-derived transferrin; and insulin of animal product-free grade, including recombinant transferrin, such as a form of transferrin made from recombinant DNA that is identical or modified to bovine, porcine or human transferrin. Preferable examples particularly include human recombinant transferrin (recombinant human transferrin), and apo transferrin, which has low iron content, is more preferable than holo transferrin, which is bound to iron. The first medium and/or the second medium for use in the present invention can be prepared by adding the transferrin as an ingredient constituting the aforementioned activin-containing supplement and/or the activin-free supplement to the aforementioned basal medium for human pluripotent stem cell, at a final concentration of 0.1 to 50 µg/mL, preferably 1 to 20 µg/mL, more preferably 2 to 10 µg/mL, and further more preferably 3 to 7 µg/mL.

Preferable examples of the aforementioned 2-mercaptoethanol include that of animal product-free grade, including chemically synthesized product produced by an ordinary method. The first medium and/or the second medium for use in the present invention can be prepared by adding the 2-mercaptoethanol as an ingredient constituting the aforementioned activin-containing supplement and/or the activin-free supplement to the aforementioned basal medium for human pluripotent stem cell, at a final concentration of 1 to 20 µM, preferably 5 to 15 µM, more preferably 7.5 to 12.5 µM, and further more preferably 9 to 11 µM.

Preferable examples of the aforementioned ethanolamine include that of animal product-free grade, including a chemically synthesized product of 2-aminoethanol, also called as monoethanolamine, produced by an ordinary method. The first medium and/or the second medium for use in the present invention can be prepared by adding the ethanolamine as an ingredient constituting the aforementioned activin-containing supplement and/or the activin-free supplement to the aforementioned basal medium for human pluripotent stem cell, at a final concentration of 1 to 20 µM, preferably 5 to 15 µM, more preferably 7.5 to 12.5 µM, and further more preferably 9 to 11 µM.

The aforementioned selenium includes selenium, derivatives thereof and salts thereof, and hydrates thereof. Examples include that of animal product-free grade, including selenic acid, sodium selenate, sodium selenite, and sodium hydrogen selenite chemically synthesized by an ordinary method. The first medium and/or the second medium for use in the present invention can be prepared by adding the selenium as an ingredient constituting the aforementioned activin-containing supplement and/or the activin-free supplement to the aforementioned basal medium for human pluripotent stem cell, at a final concentration corresponding to 1 to 40 µM, preferably 10 to 30 µM, more preferably 15 to 25 µM, and further more preferably 18 to 22 µM of sodium conversion selenate.

Examples of the aforementioned FGF-2 include basic fibroblast growth factor-2, also known as bFGF, including naturally derived FGF-2, such as porcine-derived FGF-2, bovine-derived FGF-2, or human-derived FGF-2; and FGF-2 of animal product-free grade, including genetically-modified recombinant human FGF-2, such as a form of FGF-2 made from recombinant DNA that is identical or modified to bovine, porcine or human FGF-2. Preferable examples particularly include recombinant human FGF-2 (recombinant human FGF-2 (rhFGF-2)). The first medium and/or the second medium for use in the present invention can be prepared by adding the FGF-2 as an ingredient constituting the aforementioned activin-containing supplement and/or the activin-free supplement to the aforementioned basal medium for human pluripotent stem cell, at a final concentration of 0.1 to 50 ng/mL, preferably 1 to 20 ng/mL, more preferably 3 to 7 ng/mL, and further more preferably 4 to 6 ng/mL.

Examples of the aforementioned oleic acid include plant-derived oleic acid and oleic acid chemically synthesized by an ordinary method. The first medium and/or the second medium for use in the present invention can be prepared by adding the oleic acid as an ingredient constituting the aforementioned activin-containing supplement and/or the activin-free supplement to the aforementioned basal medium for human pluripotent stem cell, at a final concentration of 0.1 to 50 µg/mL, preferably 5 to 15 µg/mL, more preferably 7.5 to 11.5 µg/mL, and further more preferably 9 to 10 µg/mL.

Examples of the aforementioned albumin include naturally derived albumin, such as egg white albumin, porcine-derived albumin, bovine-derived albumin, or human-derived albumin; and albumin of animal product-free grade, including genetically-modified albumin, such as a form of albumin made from recombinant DNA that is identical or modified to bovine, porcine or human serum albumin. Preferable example particularly include human recombinant albumin (recombinant human albumin (rHSA)). The first medium and/or the second medium for use in the present invention can be prepared by adding the albumin as an ingredient constituting the aforementioned activin-containing supplement and/or the activin-free supplement to the aforementioned basal medium for human pluripotent stem cell, at a final concentration of 0.1 to 5 mg/mL, preferably 0.5 to 2.5 mg/mL, more preferably 0.75 to 1.5 mg/mL, and further more preferably 0.9 to 1.1 mg/mL. In order to increase the solubility of the aforementioned oleic acid into medium and to increase the effect of the present invention, the albumin is preferably complexed with the aforementioned oleic acid, i.e. added as albumin-conjugated oleic acid.

Examples of the aforementioned protein kinase C inhibitor include peptides and compounds that can inhibit protein kinase C. Specific examples include a myristoylated protein kinase C peptide inhibitor (from Promega Corporation); staurosporine or staurosporine derivatives, such as calphostin, and 4'-N-benzoylstaurosporine; and bisindolyl maleimide, for example, bisindolyl maleimide I, isoquinoline sulfonyl)-2-methylpiperazine dihydrochloride (H-7), N-[2-(methylamino) ethyl] -5-isoquinoline sulfonamide (H-8), and N-(2-aminoethyl)-5-isoquinoline sulfonamide (H-9). When the aforementioned myristoylated protein kinase C peptide inhibitor (from Promega Corporation) is to be used, the proportion of human pluripotent stem cells maintained in an undifferentiated state can be increased by adding the protein kinase C peptide inhibitor as an ingredient constituting the aforementioned activin-containing supplement or the activin-free supplement to the aforementioned basal medium for human pluripotent stem cell, at a concentration of 0.5 to 5 µM, and preferably 1 to 2.5 µM.

The aforementioned activin is known to have high homology among vertebrates such as human, rat, mouse, Xenopus and zebra fish, and to have families, such as activin A, activin B, activin AB, and activin C families. Examples include activin of animal product-free grade, including genetically-modified activin, such as a form of activin made from recombinant DNA that is identical or modified to human activin. Preferable examples particularly include recombinant human activin A among the genetically-modified human activin. The first medium for use in the present invention can be prepared by adding the activin as an ingredient constituting the aforementioned activin-containing supplement to the aforementioned basal medium for human pluripotent stem cell, at a final concentration of 1 to 40 ng/mL, preferably 1 to 30 ng/mL, more preferably 1 to 10 ng/mL, and further more preferably 1 to 3 ng/mL.

The aforementioned step (a) is a step of seeding human pluripotent stem cells to the first medium comprising the aforementioned basal medium for pluripotent stem cell supplemented with the aforementioned activin-containing supplement, and culturing the cells. The pluripotent stem cells cryopreserved by the supplier are cultured preferably after the preparation by a publicly-known method. Examples of the method of preparation include a method comprising: releasing cells into Ca²⁺- and Mg²⁺-free Dulbecco phosphate-buffered saline containing EDTA, or detaching cells with trypsin, genetically-modified trypsin, trypsin/EDTA, collagenase, collagenase/trypsin, dispase, or accutase, or mechanically; collecting the released cells into the aforementioned first medium (solution) to prepare a cell suspension; precipitating and collecting cells from the cell suspension approximately at 300 to 1000 rpm; and then repeating the preparation of a cell suspension one or more times; and a method for culturing cells in a medium comprising fetal bovine serum, DMEM or DM/F12 supplemented with KSR (KnockOut Serum Replacement, from GIBCO), L-glutamine, 2-mercaptoethanol, non-essential amino acids and bFGF, and/or in the presence of sustaining cells (feeder cells) on a gelatin-coated plate. Use of the method according to the present invention has an effect that impurities that may be comprised in the aforementioned animal-derived ingredients such as fetal bovine serum, FGF, and feeder cells may be removed by subculturing for a long period of time.

The aforementioned step (b) is a step of replacing the first medium in which the human pluripotent stem cells at passage 1 are cultured with the second medium supplemented with the activin-free supplement in the first medium change, and culturing the human pluripotent stem cells. Examples of the time of medium change from the first medium to the second medium include 36 to 84 hours, preferably within 48 to 72 hours, more preferably 54 to 66 hours, and particularly preferably 57 to 63 hours after the start of culturing in the first medium. In the present invention, medium change can be conducted by an ordinary method.

The cells at passage 1 being cultured in the second medium in step (b) may be continued to be cultured with one or more times of medium change into the second medium as needed, and when the cells in culture reach the state suitable for subculturing, they are transferred and subcultured into the aforementioned first medium in step (c). Cells being cultured after the subculturing become cells at passage 2. Examples of the time for subculturing include 1 to 20 days, preferably 3 to 15 days, and more preferably 4 to 6 days after the medium change from the first medium to the second medium. Depending on the state of cells being cultured, the time for subculturing may be determined by the confluency, such as when the cells reach 55 to 95% confluent, preferably 65 to 85% confluent, and further preferably 67 to 73% confluent. Cells are also preferably subcultured when cells in the center or the marginal region of colonies start to differentiate, regardless of the confluency, and the time for subculturing may also be determined by the differentiation state of cells. Examples of the time of medium change from the second medium to the second medium include every 6 hours to 9 days, preferably every 12 hours to 5 days, more preferably every 18 hours to 2 days, and particularly preferably every 24 hours.

After these, step (b) and step (c) are repeated. Thus, the aforementioned cells at passage 2 subcultured into and cultured in the aforementioned first medium need a medium change into the second medium again in the first medium change. The human pluripotent stem cells cultured in the second medium are maintained with medium changes into the second medium, and when they are to be subcultured, they need again a step of subculturing them into the first medium, and culturing them. The subcultured cells become cells at passage 3. In the method according to the present invention, the pluripotent stem cells can be continuously cultured, while increasing the passage number one by one at the time of subculturing, and maintaining an undifferentiated state, by sequentially repeating the two steps: replacing the first medium with the second medium and subculturing into the first medium.

Examples of the method for subculturing the aforementioned human pluripotent stem cells include publicly-known methods such as a method of using trypsin, a method of using trypsin and EDTA, a method of using a mixture of trypsin, collagenase and calcium, a method of using dispase, a method of using collagenase, a method of cutting out a subpopulation of one hundred to several hundred cultured cells with a narrow tip such as a needle or a plastic Pasteur pipet under microscope. Specific examples include a method of removing the medium in the culture vessel by aspiration etc., and then treating cells with 1 unit/mL of dispase at 37°C for 1 to 10 minutes; removing the dispase; adding the first medium of the present invention, detaching a group of cells (a colony), and then conducting one to several times of centrifugation; collecting cells into the basal medium (solution) for pluripotent stem cell according to the present invention, and conducting centrifugation; dispersing cells in the basal medium solution for pluripotent stem cell; conducting centrifuge again; and dispersing cells in the first medium.

The pluripotent stem cells to be cultured by the method according to the present invention are preferably cultured at 33 to 40°C, preferably 34 to 39°C, and particularly preferably 37°C, in the presence of 1 to 20%, preferably 3 to 15%, and particularly preferably 8 to 10% of carbon dioxide, in a high humidity of 75% or more, preferably 85% or more, more preferably 95%, and particularly preferably 100%, in any of the aforementioned steps.

Examples of the method of determining whether cells cultured by the method according to the present invention are cells maintaining an undifferentiated state or not include a method of determination by detection of the expression of undifferentiation markers and/or nondetection of the expression of differentiation markers with antibodies against various markers (proteins); a method of determination by detection of the expression of various markers (genes); a method of observing morphological characteristics of cells; and a method of determining whether the cells can differentiate into specific cells in the response to the stimulation with a specific differentiation-inducing factor.

In the method of determination using the aforementioned markers, when undifferentiation markers such as SSEA-3, SSEA-4, TRA-2-54, TRA-1-60, TRA-1-80, CD90, Nanog, Oct-3, Oct-4, and alkaline phosphatase are expressed, then the cells cultured by the method according to the present invention can be determined to have maintained an undifferentiated state; and when differentiation markers such as SSEA-1, CD105, CD56, A2B5 are not expressed, then the cells cultured by the method according to the present invention can also be determined to have maintained an undifferentiated state.

The expression of the aforementioned undifferentiation and/or differentiation markers at protein level can be determined by flow cytometry, immunostaining, ELISA, or the like with specific antibodies against respective markers; and the expression of the aforementioned markers at gene level can be determined by RT-PCR with specific primer pairs, Northern blotting or the like for respective marker genes with specific probes.

Examples of the method of determining whether the markers are expressed or not by the aforementioned flow cytometry include a method of determination by treating the cells cultured by the method according to the present invention with trypsin in PBS solution comprising trypsin/EDTA; suspending the treated cells into 1 mL of 10% goat serum for 30 minutes, and then conducting centrifuge; incubating the cells with anti-mouse antibodies against the target marker proteins for 30 hours, and then washing the aforementioned cultured cells three times with PBS containing 1% goat serum; allowing the cells to react with AlexaFluor-conjugated anti-mouse goat IgG antibodies for 30 minutes; washing the cells three times with PBS containing 1% goat serum; and examining the resuspended cells with an appropriate flow cytometry apparatus.

Examples of the method of determining whether the markers are expressed or not by the aforementioned immunostaining include a method of determination by fixing the cells cultured by the method according to the present invention with 4% paraformaldehyde (PFA) in PBS, and then washing the cells multiple times with PBS; increasing the permeability of the cultured cells with Triton X or the like; conducting the blocking with 10% goat serum and then the immunostaining with mouse antibodies against the target marker proteins; allowing the cells to react with AlexaFluor-labeled anti-mouse goat IgG; and observing through fluorescent microscope.

Examples of the method of determining whether the aforementioned alkaline phosphatase is expressed or not include a method of so-called alkaline phosphatase staining, comprising fixing the cells cultured by the method according to the present invention with 4.5 mM citric acid, 2.25 mM sodium citrate, 3 mM sodium chloride, 65% methanol and 4% paraformaldehyde for 5 minutes; washing the cells; and then visualizing alkaline phosphatase with a suitable kit such as FastRed substrate kit (from Sigma Chemical Co.).

Examples of the methods of determination by observing morphological characteristics of the aforementioned cells include a method of determining whether an undifferentiated state is maintained based on the following indexes: the border between cells being unclear and cells being mostly occupied with the nucleus with little cytoplasm; populations of cells (colonies) having clear circular outlines; many colonies being slightly piled-up with cells; and the absence of fibroblast-like cells or nerve-like cells emerging from the border of colony.

Examples of the aforementioned method of determining whether the cells cultured by the method according to the present invention have maintained an undifferentiated state or not by determining whether the cells can differentiate into specific cells in the response to the stimulation with a specific differentiation-inducing factor include a method comprising determining that the cells have maintained an undifferentiated state if the aforementioned cells differentiate into, for example, epithelium-like cells when bone morphogenetic protein 4 (BMP4) is added to the medium.

The culture vessel for use in the method for maintaining and culturing according to the present invention is not particularly limited, as far as it is a vessel that allows to maintain and culture pluripotent stem cells for use in the present invention. Examples include flasks, flasks for tissue culture, dishes, Petri dishes, dishes for tissue culture, multi-dishes, microplates, microwell plates, laboratory dishes, tubes, trays, and culture bags.

The present invention provides a kit for culturing a human pluripotent stem cell while maintaining an undifferentiated state. The kit according to the present invention comprises an activin-containing supplement, an activin-free supplement and an ingredient of basal medium for human pluripotent stem cell. The ingredients constituting the aforementioned respective compositions also comprise those in which a part or all the ingredients are wrapped individually, and those in which two or more compositions are mixed and wrapped. The aforementioned kit for culturing a human pluripotent stem cell can further comprise an antibody against an undifferentiation and/or differentiation marker protein, or a primer or a probe to detect an undifferentiation and/or differentiation marker gene to determine the undifferentiated state of human pluripotent stem cells.

Hereinafter, the present invention will be described in further detail with reference to Examples, but the present invention is not in any manner limited to such specific Examples. The ES cell lines and iPS cell lines, which are pluripotent stem cells used in Examples, were obtained from distributing institutes and used in experiments following the governmental guidance. Other cell lines are also stored at National Institute of Biomedical Innovation and distributable under certain conditions.

### Examples

### Example 1

### [Study on supplement]

### (Basal medium)

Studies on substituting conventional animal-derived materials with animal-free products were conducted. Used was hESF9 medium shown in Table 2 below as a conventional medium.

**[Table 2]**

| Composition of hESF9 Medium | | | |
|---|---|---|---|
| | mg/L | | mg/L |
| Calcium chloride | 108 | Glycine | 19.375 |
| Calcium nitrate | 25 | L-Alanine | 2.225 |
| CuSO₄ | 0.000625 | L-Arginine hydrochloride | 94.75 |
| Ferric Nitrate (Fe(NO₃)₃) | 0.005 | L-Asparagine H₂O | 16.2525 |
| Ferric sulfate (FeSO₄·7H₂O) | 0.2085 | L-Aspartic Acid | 8.325 |
| Magnesium Chloride(anhydrous) | 30.515 | L-Cystelne HCl·H₂O | 7.88 |
| Magnesium Sulfate(anhydrous) | 61.055 | L-Cystine 2HCl | 47.5725 |
| Potassium chloride (KCl) | 355.9 | L-Glutamic Acid | 8.675 |
| Sodium bicarbonate(NaHCO₃) | 2000 | L-Glutamine | 549.65 |
| Sodium chloride (NaCl) | 6599.75 | L-Histidine | 23.165 |
| Sodium phosphate (NaH₂PO₄) (anhydrous) | 62.5 | L-Isoleucine | 65.935 |
| Sodium phosphate, dibas(Anhydrous) | 235.51 | L-Leucine | 68.225 |
| Sodium selenite | 0.0034588 | L-Lysine hydrochloride | 92. 175 |
| Zinc sulfate | 0.216 | L-Methionine | 19.87 |
| Biotin | 0.05185 | L-Phenylalanine | 37.99 |
| Ethanolamine | 0.6108 | L-Proline | 13.625 |
| Lipoic Acid | 0.0525 | L-Serine | 31.125 |
| Linoleic Acid | 0.021 | L-Threonine | 55.525 |
| Oleic Acid | 9.4 | L-Tryptophan | 9.76 |
| Ascorbic acid 2-phosphate | 100 | L-Valine | 54. 825 |
| B12 | 0.34125 | L-arginine | 50 |
| Choline Chloride | 6.24 | L-tyrosin | 42.36 |
| Folic Acid | 2.575 | L-hydroxyprolin | 5 |
| Myo-Inositol | 16.85 | Albumin, bovine | 1000 |
| Niacinamide | 2.25925 | bFGF | 0.01 |
| Pantothenic acid | 2.1825 | Glutathione | 0.25 |
| Pyridoxal hydrochloride | 2 | Heparin sulfate sodium salt | 0.1 |
| Pyridoxine hydrochloride | 0.2655 | Transferrin (human-derived) | 5 |
| Riboflavin | 0.2595 | Hypoxanthine | 1. 02 |
| Thiamine hydrochloride | 2.335 | Insulin Recombinant Full Chain | 10 |
| Glucose | 2500 | 2-mercaptoethanol | 0. 7813 |
| Sodium Pyruvate | 110 | Kanamycin sulfate | 100 |
| | | Phenol Red (Sodium) | 6.56 |
| | | Putrescine-2HCl | 0.04025 |
| | | p-Aminobenzoic acid | 0.25 |
| | | Thymidine | 0.1825 |

### [Supplement ingredient; study on fibronectin]

The inventors studied on the substitution of ECMs such as collagen I, gelatin, laminin, fibronectin, and the like, which are recently used as a cell support in place of feeder cells, from animal-derived materials to animal product-free ingredients. Studies were conducted with human ES cell line KhES-1 (Laboratory of embryonic stem Cell research Center, Institute for Frontier Medical Sciences, Kyoto University), and human iPS cell line Tic (JCRB1331) (JSCB cell bank, National Institute of Biomedical Innovation); and the embryonal cancer cell line PA-1 (JCRB9061) (JSCB cell bank, National Institute of Biomedical Innovation) was used as a comparative example.

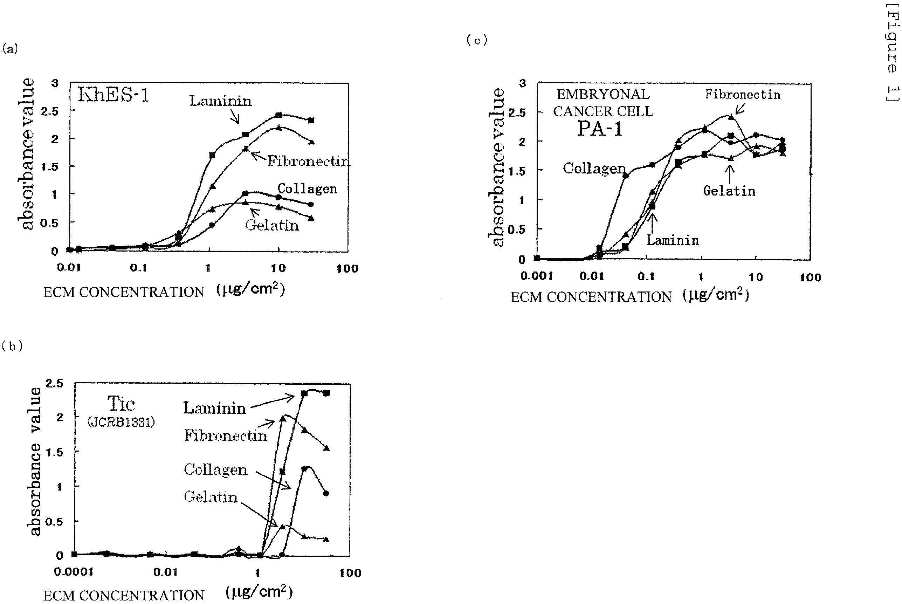

Using 96 well microplates (from Corning Incorporated), each of collagen I (from Nitta Gelatin Inc.), gelatin (from Sigma Chemical Co.), laminin (from Sigma Chemical Co.), and fibronectin (from Sigma Chemical Co.) was treated at 37°C for 3 hours, with avoiding being dried, to coat the microplates with each at 0.01, 0.05, 0.1, 0.5, 1, 5 and 10 µg/cm². The aforementioned hESF9 medium was added, and then human ES cell line KhES-1 and human iPS cell line Tic (JCRB1331), and embryonal cancer cell line PA-1 (JCRB9061) were seeded at 3 x 10⁶ cells/cm², after counting their cell numbers. After culturing the cells for two days (or culturing the embryonal cancer cell line PA-1 (JCRB9061) for one hour), the cells were fixed and nuclei of adhered cells were stained with 0.4% crystal violet (from Sigma Chemical Co.) dissolved in methanol for 30 minutes. The microplates were washed and then dried. The cells were lysed with 0.1 M sodium citrate dissolved in 50% methanol, and absorbance at 595 nm was measured with a microplate reader (model 550, from Bio-Rad Laboratories, Inc.). The results are shown in Figures 1(a) to 1(c).

### (Results)

Human ES cell line KhES-1 had the strongest adhesion ability to laminin and fibronectin, and weak adhesion ability to collagen and gelatin regardless of their concentrations (see Figure 1(a)). Human iPS cell line Tic (JCRB1331) had the strongest adhesion ability to laminin at about 10 to 30 µg/cm² in concentration, and to fibronectin at about 3 to 9 µg/cm² in concentration, and weak adhesion ability to collagen and gelatin regardless of their concentrations (see Figure 1(b)).

### (Comparative example)

Embryonal cancer cell line PA-1 (JCRB9061) was studied on the adhesion ability to the various ECMs described above. PA-1 had the strongest adhesion ability to collagen and gelatin at about 10 µg/cm² in concentration (see Figure 1(c)).

### (Discussion)

The above results showed that the pluripotent stem cells have strong adhesion ability to laminin and fibronectin. Cells cultured on laminin, however, express cytokeratin strongly. Therefore, bovine- or human-derived fibronectin was determined to be used as an ingredient of the supplements according to the present invention.

### [Supplement ingredient: study on substituting ingredient for bovine albumin-conjugated oleic acid]

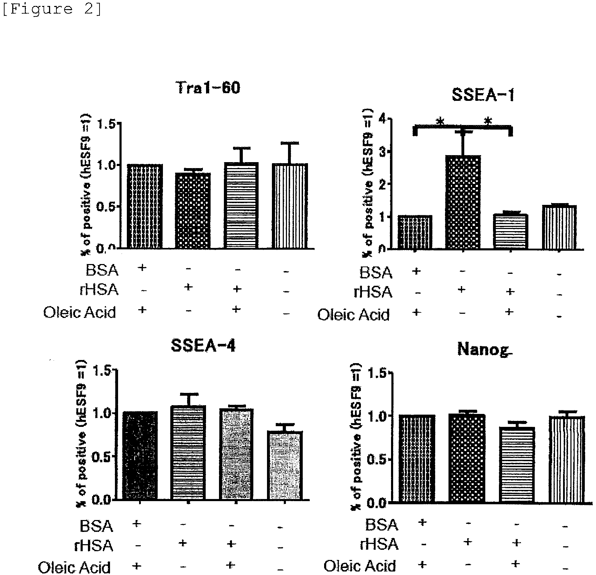

Bovine albumin (bovine serum albumin: BSA) has been used in many conventional media such as the aforementioned hESF9 medium. Since it is an animal-derived material, its substitution with an animal product-free ingredient was studied. The previous studies of the inventors have revealed that cell proliferation and the maintenance of an undifferentiated state tend to be better at concentrations of 9.4 µg/mL oleic acid with 1 mg/mL albumin (from Sigma Chemical Co.). Using the undifferentiation markers SSEA-4, TRA-1-60, and Nanog, and the differentiation marker SSEA-1, effects of adding bovine albumin or recombinant human albumin (rHSA, from Millipore Corporation) and/or oleic acid on the maintenance of an undifferentiated state of the ES cells were studied. When adding oleic acid and albumin, they were added in the form of albumin-conjugated oleic acid. Among the ingredients of the hESF9 medium, BSA and oleic acid were substituted with BSA-conjugated oleic acid (medium condition 1), rHSA alone (medium condition 2), or rHSA-conjugated oleic acid (medium condition 3). A medium in which none of BSA, rHSA, or oleic acid was added to the hESF9 medium was used as a negative control (medium condition 4). Cell line H9 (WA09) was cultured under these conditions. Cell line H9 (WA09) being cultured was cultured in hESF9 medium for 1 passage, subjected to fluorescent immunostaining, and analyzed on the percentage of positive cells with expression. The cells were fixed with 4% paraformaldehyde (PFA) in PBS at room temperature for 15 minutes, blocked with bovine serum, and then immunostained using anti-SSEA-4 antibody (from Abcam plc.) and AlexaFluor-labeled anti-mouse IgG (from Invitrogen Corporation); anti-TRA-1-60 antibody (from Abcam plc.) and AlexaFluor-labeled anti-mouse IgM (from Invitrogen Corporation); or anti-SSEA-1 antibody (from Abcam plc.) and AlexaFluor-labeled anti-mouse IgM (from Invitrogen Corporation). After the blocking with bovine serum comprising Triton X, the cells were treated with immunostaining with anti-Nanog antibody (from Cell Signaling Technology, Inc.) and anti-OCT4 antibody (from Santa Cruz Biotechnology, Inc.), reacted with an AlexaFluor-labeled secondary antibody, and observed under fluorescent microscope. Details of the medium conditions described above are shown in Table 3, and the results are shown in Figure 2.

**[Table 3]**

| | BSA | rHSA | Oleic acid | |
|---|---|---|---|---|
| Medium condition (1) | + | - | + | BSA-conjugated oleic acid (known hESF9 medium) |
| Medium condition (2) | - | + | - | Recombinant human albumin (rHSA) |
| Medium condition (3) | - | + | + | rHSA-conjugated oleic acid |
| Medium condition (4) | - | - | - | None |

### (Result)

There were no significant differences between the medium conditions (1) to (4) in the percentage of positive cells expressing the undifferentiation marker SSEA-4, TRA-1-60, or Nanog in cell line H9 (WA09), among the cells cultured for 1 passage (see Figure 2). On the other hand, the percentage of positive cells expressing the differentiation marker SSEA-1 was found to be significantly (* mark) increased in the medium condition (2), in which rHSA alone was added. The foregoing results indicated that though rHSA does not affect the expression of undifferentiation markers, addition of rHSA conjugated with oleic acid decreases the expression of differentiation markers, and has an effect of suppressing the differentiation.

### (Fluorescent immunostaining)

Under the medium condition (3), cultured cells of cell line H9 (WA09) at passage 3 were examined with fluorescent immunostaining using the undifferentiation markers SSEA-4, Tra1-60, and Nanog, and the differentiation marker SSEA-1.

### (Result)

The cultured cells of cell line H9 (WA09) immunostained with antibodies against the undifferentiation markers SSEA-4, Tra1-60, and Nanog were found to show high expression of the antibodies, but low expression of the differentiation marker SSEA-1. The foregoing results confirmed that supplements comprising rHSA conjugated with oleic acid are effective for maintaining the undifferentiated state of pluripotent stem cells.

### [Supplement ingredient; removal of porcine-derived heparin]

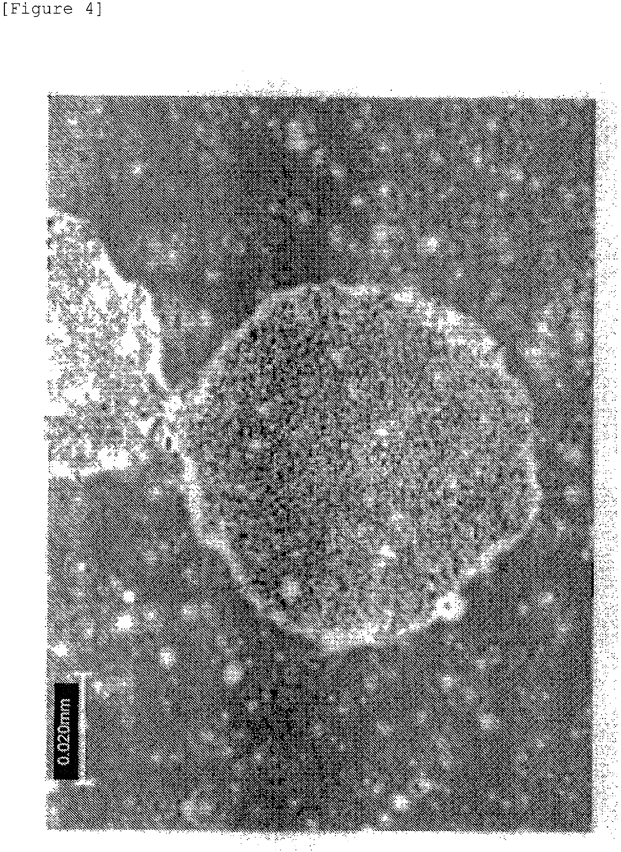

Previously, the present inventors developed a medium comprising heparin for maintaining primate embryonic-stem cells (see for example, Japanese unexamined Patent Application Publication (Translation of PCT Application) No. 2009-542247, described above). Since most of sources of heparin are animals such as pig, the present inventors tried culturing cell line H9 (WA09) in a hESF-9 medium lacking heparin and supplemented with activin. Observation of cultured cells of cell line H9 (WA09) at passage 2, stained with anti-Tra-1-60 antibody under microscope revealed characteristics such as cellular morphologies with the border between cells being unclear and cytoplasm being mostly occupied with the nucleus; colonies with circular outlines; and colonies being slightly piled-up with cells (see, Figure 4). These confirmed that the pluripotency of the cells was maintained. Based on these results, porcine-derived heparin was determined to be removed from the medium ingredients.

### Example 2

### [Basal medium; preparation of hESF-grow medium]

The inventors used to add HEPES to conventional media (see for example, international publication No. WO2005/063968 pamphlet, described above). HEPES was, however, removed based on the finding that addition of HEPES to serum-free medium has a very adverse effect on the maintenance of human ES cells (see for example, PNAS, vol.105, no. 36, 13409-13414, described above). Based on the finding that an undifferentiated state is maintained by addition of ascorbic acid (Japanese unexamined Patent Application Publication (Translation of PCT Application) No. 2009-542247), a basal medium of the composition shown in Table 4 below (hESF-grow medium) was prepared as an animal product-free basal medium and sterilized according to an ordinary method.

**[Table 4]**

| hESF-grow medium | | | |
|---|---|---|---|
| INORGANIC SALTS | mg/L | VITAMINS | mg/L |
| CaCl₂ | 108.305 | Ascorbic acid 2-phosphate | 100 |
| Ca (NO₃)₂ | 25 | D-Biotin | 0.05185 |
| CuSO₄·5H₂O | 0.000625 | Choline Chloride | 6.24 |
| Fe(NO₃)₃·9H₂O | 0.05 | Folic Acid | 2.575 |
| FeSO₄·7H₂O | 0.2085 | I-Inositol | 16.85 |
| MgCl·6H₂O | 30.515 | Niacinamide | 2.25925 |
| MgSO₄ | 61.055 | D-Pantothenic Acid·½Ca | 2.1825 |
| KCl | 355.9 | Pyridoxal·HCl | 2 |
| NaCl | 6599.75 | Pyridoxine·HCl | 0.2655 |
| Na₂HPO₄ 2H₂O | 295.28 | Riboflavin | 0.2595 |
| NaH₂PO₄ H₂O | 61.51 | Thiamine·HCl | 2.335 |
| ZnSO₄·7H₂O | 0.216 | Vitamin B-12 | 0.34125 |
| AMINO ACIDS | mg/L | p-aminobenzoic acid | 0.25 |
| L-Alanine | 2.225 | | |
| L-Arginine | 50 | MISC. | mg/L |
| L-Arginine·HCl | 94.75 | NaHCO₃ | 2000 |
| L-Asparagine·H₂O | 16.2525 | Gluthathione | 0.25 |
| L-Aspartic Acid | 8.325 | Thymidine | 0.1825 |
| L-Cystine·HCl·H₂O | 47.5725 | Hypoxanthine | 1.02 |
| L-Cysteine·2HCl | 7.88 | Lipoic acid | 0.0525 |
| L-Glutamic Acid | 8.675 | Linoleic acid | 0.021 |
| L-Glutamine | 549.65 | Phenol Red·Na | 6.56 |
| Glycine | 19.375 | Putrescine·2HCl | 0.04025 |
| L-Histidine-HCl· | 23.165 | Pyruvic acid·Na | 110 |
| L-Hydroxyproline | 5 | | |
| L-Isoleucine | 65.935 | D-Glucose | 2000 |
| L-Leucine | 68.225 | | |
| L-Lysine·HCl | 92.175 | | |
| L-Methionine | 19.87 | | |
| L-Phenylalanine | 37.99 | | |
| L-Proline | 13.625 | | |
| L-Serine | 31.125 | | |
| L-Threonine | 55.525 | | |
| L-Tryptophan | 9.76 | | |
| L-Tyrosine· | 42.36 | | |
| L-Valine | 54.825 | | |

Based on the findings of the inventors and the foregoing studies, the inventors tried culturing pluripotent stem cells using a medium prepared by adding to the aforementioned hESF-grow medium a supplement to be used for culturing human pluripotent stem cells while maintaining an undifferentiated state, which supplement comprises a suitable combination of bovine fibronectin, recombinant human insulin, recombinant human transferrin (apo), 2-mercaptoethanol, 2-ethanolamine, sodium selenate, recombinant human FGF-2, recombinant human albumin-conjugated oleic acid, and recombinant human activin.

In the following studies, cryopreserved cell line H9 (WA09) was subjected to a preparation prior to its seeding, by seeding mouse embryonic fibroblasts (from Millipore Corporation) whose cell growth is arrested with mitomycin C as feeder cells in Dulbecco's modified Eagle medium comprising 10% fetal bovine serum in a flask (from Corning Incorporated) coated with 0.1% gelatin (Embryomax, from Millipore Corporation); adding DM/F12 (from GIBCO) supplemented with 1 mM L-glutamine (21-51-016, from GIBCO), 0.1 mM 2-mercaptoethanol, 1% non-essential amino acids (11140-035, from GIBCO), 20% KSR (10828-028, from GIBCO) and 4 ng/mL bFGF (13256-029, from KATAYAMA CHEMICAL INDUSTRIES Co., Ltd.); and maintaining the cells in the resulted KSR-addition medium.

### (Preparation of medium 1)

A solution of (1) human plasma fibronectin (F0895, from Sigma Chemical Co.) dissolved in PBS at 2 µg/cm² was transferred into a 25 cm² flask (from Corning Incorporated). The flask was treated at 37°C for 3 hours, with avoiding being dried, and the solution was aspirated just prior to seeding cells. Activin-containing supplement + hESF-grow medium was prepared on the coating in the flask by adding the hESF-grow medium supplemented with a supplement of 8 factors of (1) to (8) and 2 ng/mL activin (N-338 AC/CF, from R&D Systems, Inc.), as an activin-containing supplement: (2) 10 µg/mL recombinant human insulin (19278-5ML, from Sigma Chemical Co.); (3) 5 µg/mL recombinant human transferrin (apo) (T 2252, from Sigma Chemical Co.); (4) 10 µM 2-mercaptoethanol (M 7522, from Sigma Chemical Co.); (5) 10 µM 2-ethanolamine (E 0135, from Sigma Chemical Co.); (6) 20 nM sodium selenite (S 9133, from Sigma Chemical Co.); (7) rHSA-conjugated oleic acid wherein 9.4 µg/mL oleic acid (O-1383-5G, from Sigma Chemical Co.) is complexed with 1 mg/mL recombinant human albumin; (8) 5 ng/mL basic FGF (from KATAYAMA CHEMICAL INDUSTRIES Co., Ltd.).

### (Preparation of medium 2)

Activin-free supplement + hESF-grow medium was prepared by adding a supplement of 8 factors of (1) to (8) described above, as an activin-free supplement, to the hESF-grow medium described above.

### (Culturing in activin-containing medium)

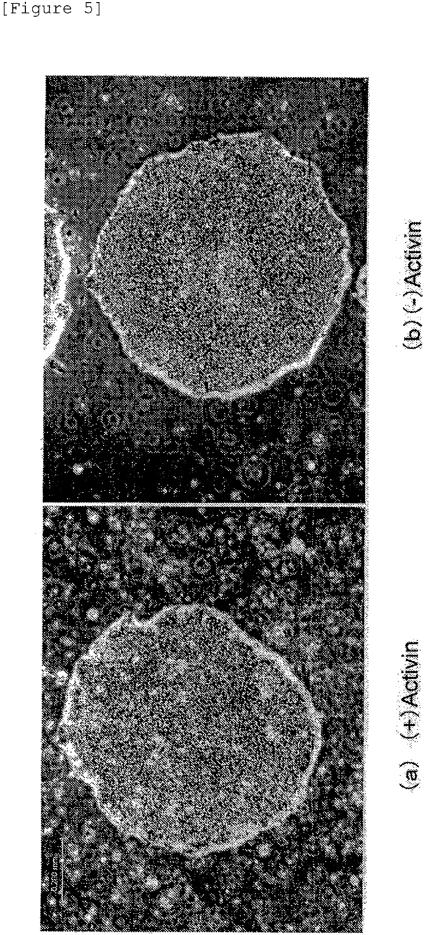

Cell line H9 (WA09) was cultured in the activin-containing supplement + hESF-grow medium. 60 hours after starting the culturing, a medium change into the activin-containing supplement + hESF-grow medium was conducted. 7 days after starting the culturing, the cells were observed. A micrograph of the cells is shown in Figure 5(a). The colony is in a flat form and with a deformed shape, confirming that the cells likely have lost the undifferentiated state.

### (Medium change into activin-free medium)

Cell line H9 (WA09) was cultured in the activin-containing supplement + hESF-grow medium. 60 hours after starting the culturing, a medium change into the activin-free supplement + hESF-grow medium was conducted. 5 days after starting the culturing, the cells were observed. A micrograph of the cells is shown in Figure 5(b). The colony is in a piled-up form and with a round shape, confirming that the cells likely have not lost the undifferentiated state.

### Example 3

### [Culturing human pluripotent stem cells while maintaining undifferentiated state]

### (Fibronectin coating)

After this, studies were continued using the activin-containing supplement + hESF-grow medium as the first medium, and the activin-free supplement + hESF-grow medium as the second medium.

Cell line H9 (WA09) was maintained in the KSR-addition medium. After removing the medium and treating the cells with 1 unit/mL dispase at 37°C for 2 minutes, dispase was removed, the cell population was collected using a scraper or the like with the first medium solution according to the present invention. After centrifugation at 300 rpm for 1 minute, the cells were dispersed in the first medium solution and subjected to centrifugation again. The cells were then dispersed in the first medium to start culturing at passage 1.

Cell line H9 (WA09) was cultured in the first medium. 60 hours after starting the culturing, a medium change into the second medium was conducted. Moreover, three times of medium change into the second medium were conducted every 24 hours. Three days after the medium change from the first medium to the second medium, the medium was removed from the culture of cell line H9 (WA09). After treating the cells with 1 unit/mL dispase at 37°C for 2 minutes, dispase was removed, the cell population was collected using a scraper or the like with the first medium solution according to the present invention. After centrifugation at 300 rpm, the cells were dispersed in the first medium solution and subjected to centrifugation again. The cells were dispersed in the first medium to be transferred (subcultured), and cultured at passage 2. When the timing of use of the cells used at passage 1 was optimum, passage 2 starts at the third or fourth day, and, thereafter, subculturing is at the fifth day.

The cell line H9 (WA09) at passage 2 subcultured into the first medium was cultured in the first medium. 60 hours after starting the culturing, a medium change into the second medium was conducted. 5 times of medium change into the second medium were conducted, and then on the fifth day after the medium change from the first medium to the second medium, the cells were subcultured again into the first medium and cultured at passage 3. After this, the steps of subculturing cells into the first medium and changing medium to the second medium were repeated, and culturing was continued.

### (Morphological observation)

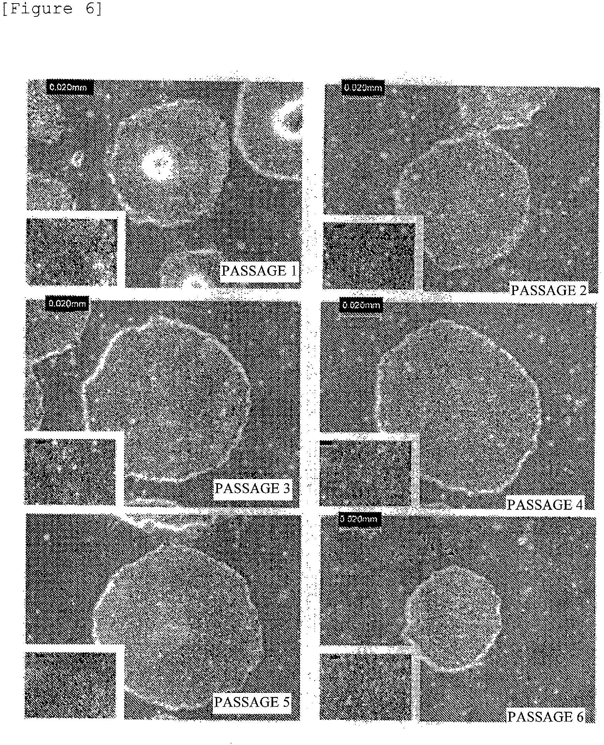

Figure 6 is a series of photographs showing the phase contrast microscope observation of cells at passage 1 to 6 of the cultured cells of the cell line H9 (WA09) cultured by the aforementioned method. At any period, the characteristics of undifferentiated cells: the border between cells being unclear, and cells being mostly occupied with the nucleus with little cytoplasm; populations of cells (colonies) having clear circular outlines; many colonies being slightly piled-up with cells; and the absence of fibroblast-like cells or nervelike cells emerging from the border of colony, were observed, confirming that the pluripotency was maintained.

### (Analysis by flow cytometry)

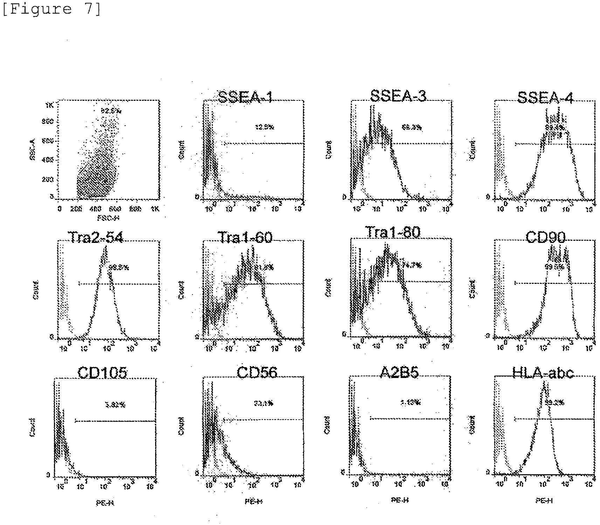

Cell line H9 (WA09) was analyzed by flow cytometry on the undifferentiated state of the cultured cells of the cell line H9 (WA09) cultured by the aforementioned method, using SSEA-3, SSEA-4, TRA-2-54, TRA-1-60, TRA-1-80 and CD90 (from Becton, Dickinson and Company) as undifferentiation markers, and SSEA-1, CD105 (from Becton, Dickinson and Company), CD56 (from Becton, Dickinson and Company) and A2B5 (from Millipore Corporation) as differentiation markers. HLA-abc (from Becton, Dickinson and Company) was used to confirm that cells are derived from human. The cultured cells of the cell line H9 (WA09) at passage 5 were treated with PBS solution comprising trypsin/EDTA, and dispersed on the fifth day after the last transfer. The cells were suspended in 1 mL of 10% bovine serum (from HyClone Laboratories, Inc.) for 30 minutes, centrifuged, and then incubated with mouse antibodies (from Abcam plc.) against respective markers for 30 minutes. The cultured cells were washed three times with PBS containing 1% bovine serum, and reacted with AlexaFluor-conjugated anti-rat IgM, goat anti-mouse IgG, and IgM antibody (from Invitrogen Corporation) for 15 minutes. The cultured cells were washed three times with PBS containing 1% bovine serum, then resuspended, and analyzed with BD FACSCanto (TM) flow cytometer (from Becton, Dickinson and Company, NJ USA). The results are shown in Figure 7.

### (Result)

As apparent from Figure 7, cells highly expressing the undifferentiation marker SSEA-3, SSEA-4, TRA-2-54, TRA-1-60, TRA-1-80 and CD90 were shown to be 66.3 to 99.5%. Expression of the differentiation marker SSEA-1, CD105, CD56, or A2B5 was 1.12 to 12.5%. HLA-abc was 99%, indicating that they are human-derived cells. These results indicated that the cells of cell line H9 (WA09) cultured on the fibronectin coating were pluripotent stem cells maintaining the undifferentiated state even after 5 passages.

### Example 4

### [Culturing human pluripotent stem cells while maintaining undifferentiated state]

### (Preparation of first medium containing fibronectin)

In a 6 well plate (from Becton, Dickinson and Company), first media containing fibronectin were prepared by adding 10 µg/mL recombinant human insulin, 5 µg/mL recombinant human transferrin (apo), 10 µM 2-mercaptoethanol, 10 µM 2-ethanolamine, 20 nM sodium selenite, 9.4 µg/mL oleic acid complexed with 1 mg/mL recombinant human albumin, and 2 ng/mL recombinant human activin to the hESF-grow medium, and further adding 0, 20, 40, 80, 160, or 320 µg/well of bovine fibronectin to each well.

### (Preparation of second medium containing fibronectin)

In a 6 well plate (from Becton, Dickinson and Company), second media containing fibronectin were prepared by adding 5 µg/mL recombinant human transferrin (apo), 10 µM 2-mercaptoethanol, 10 µM 2-ethanolamine, 20 nM sodium selenate and 9.4 µg/mL oleic acid complexed with 1 mg/mL recombinant albumin to the hESF-grow medium, and further adding 0, 20, 40, 80, 160, or 320 µg/well of bovine fibronectin to each well.

The cell line H9 (WA09) was cultured similarly to the culturing on fibronectin coating described above, except that the first medium and the second medium described in the above section on fibronectin coating were replaced with the first media containing fibronectin and the second media containing fibronectin, and cells were cultured at varying concentrations of fibronectin in respective wells. The area of 1 well in the used plate was 9.6 cm², and 3 mL each of the medium was added.

### (Result)

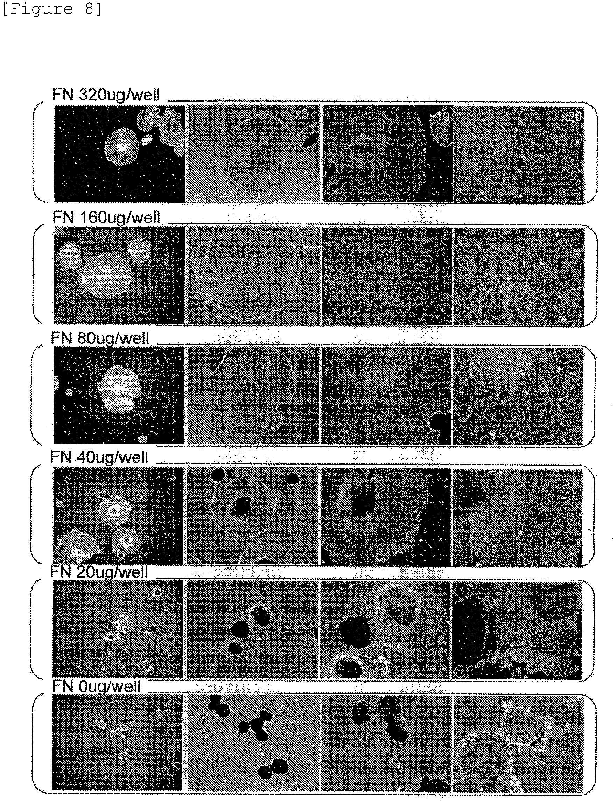

Micrographs of the cultured cells of cell line H9 (WA09) after subculturing into a serum-free medium are shown in Figure 8. Based on the appearance of the cells, the undifferentiated state was found to be best maintained when supplemented with 80 µg/well of fibronectin. Fibronectin was confirmed to be effective in the method according to the present invention, even when added to a basal medium with other supplement ingredients, besides applying to the culture vessel as a coating. These results confirmed that by using the culture method according to the present invention, human pluripotent stem cells maintain the undifferentiated state even after repeated subculturing.

### Example 5

### [Culturing human ips cells while maintaining undifferentiated state]

### (Culturing with medium containing protein kinase C peptide inhibitor)

Media comprising the activin-containing supplement + hESF-grow medium or the activin-free supplement + hESF-grow medium with fibronectin coating described in Example 3 and 0, 1, 2.5, 5, or 10 µM each of myristoylated protein kinase C peptide inhibitor (Myr. RFARKGALRQKNV) (from Promega Corporation) added thereto were prepared, and used as first media containing a protein kinase C inhibitor and second media containing a protein kinase C inhibitor.

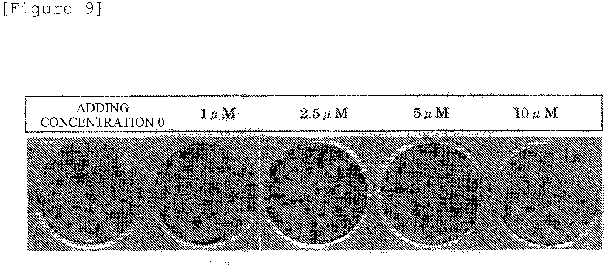

Human iPS cell line Tic (JCRB1331) was dispersed in the first medium containing a protein kinase C inhibitor and culturing was started. 60 hours after starting the culturing of the cell line Tic, a medium change into the second medium containing a protein kinase C inhibitor was conducted. Twice of medium changes were further conducted every 24 hours. On the fourth day after starting the culturing, the cells were fixed with 4.5 mM citric acid, 2.25 mM sodium citrate, 3 mM sodium chloride, 65% methanol and 4% paraformaldehyde for five minutes. Alkaline phosphatase staining was conducted with FastRed substrate kit (from Sigma Chemical Co.) according to the guidance of the manufacturer. The results are shown in Figure 9.

### (Result)

Photographs of the cell line Tic on the fourth day after starting the culturing are shown in Figure 9. When cultured in a medium comprising 1 to 5 µM of myristoylated protein kinase C (PKC) peptide inhibitor, further suppression of differentiation was observed, compared to the suppression when no inhibitor was added. Moreover, when 1 to 2.5 µM was added to the medium, increase in proliferation was observed.

### Industrial Availability

Recently, much attention has been attracted to regenerative medicine as a therapy that compensates for shortcomings of conventional organ transplantation. In regenerative medicine, a tissue or organ is generated to compensate for deficient tissue by artificially inducing the differentiation of stem cells, which are thought to have an ability to differentiate in multiple directions. According to the method of the present invention, it becomes possible to provide pluripotent stem cells maintaining an undifferentiated state, and to apply them to regenerative medicine to build tissues and organs of interest in vitro or in vivo, thereby solving various problems involved in the transplantation treatment including autologous transplantation.

## Claims

1. A method for culturing a human pluripotent stem cell while maintaining an undifferentiated state, comprising the steps (a) to (c), wherein after the step (a), the steps (b) and (C) are sequentially repeated:
(a) a step of culturing a human pluripotent stem cell in a first medium comprising a basal medium for pluripotent stem cell supplemented with an activin-containing supplement;
(b) a step of replacing the first medium with a second medium comprising the basal medium for pluripotent stem cell supplemented with an activin-free supplement, and culturing the human pluripotent stem cell; and
(c) a step of subculturing the human pluripotent stem cell into the first medium.

2. The method for culturing according to claim 1, wherein the supplements comprise fibronectin, insulin, transferrin, 2-mercaptoethanol, 2-ethanolamine, sodium selenite and an albumin-conjugated oleic acid.

3. The method according to claim 2, wherein fibronectin is used without being coated on an inside of a culture vessel.

4. The method according to claim 2 or 3, wherein the supplements further comprise a protein kinase C inhibitor.

5. The method for culturing according to any one of claims 1 to 4, wherein the basal medium is a chemically defined medium comprising one or more sugars, one or more inorganic salts, one or more amino acids, one or more vitamins and a trace ingredient.

6. The method for culturing according to any one of claims 1 to 4, wherein the basal medium is a hESF-grow medium shown in Table 1:
**[Table 1]**
| hESF-grow medium | | | |
|---|---|---|---|
| INORGANIC SALTS | mg/L | VITAMINS | mg/L |
| CaCl₃ | 108.305 | Ascorbic acid 2-phosphate | 100 |
| Ca(NO₃)₂ | 25 | D-Biotin | 0.05185 |
| CuSO₃·5H₂O | 0.000625 | Choline Chloride | 6.24 |
| Fe(NO₃)₃·9H₂O | 0.05 | Folic Acid | 2.575 |
| FeSO₄·7H₂O | 0.2085 | I-Inositol | 16.85 |
| MgCl·6H₂O | 30.515 | Niacinamide | 2.25925 |
| MgSO₄ | 61.055 | D-Panthothenic Acid·½Ca | 2.1825 |
| KCl | 355.9 | Pyridoxal·HCl | 2 |
| NaCl | 6599.75 | Pyridoxine·HCl | 0.2655 |
| Na₂HPO₄ 2H₂O | 295.28 | Riboflavin | 0.2595 |
| NaH₂PO₄ H₂O | 61.51 | Thiamine·HCl | 2.335 |
| ZnSO₄·7H₂O | 0.216 | Vitamin B-12 | 0.34125 |
| AMINO ACIDS | mg/L | p-aminobenzoic acid | 0.25 |
| L-Alanine | 2.225 | | |
| L-Arginine | 50 | MISC. | mg/L |
| L-Arginine·HCl | 94.75 | NaHCO₃ | 2000 |
| L-Asparagine·H₂O | 16.2525 | Gluthathione | 0.25 |
| L-Aspartic Acid | 8-325 | Thymidine | 0.1825 |
| L-Cystine·HCl·H₂O | 47.5725 | Hypoxanthine | 1.02 |
| L-Cysteine·2HCl | 7.88 | Lipoic acid | 0.0525 |
| L-Glutamic Acid | 8.675 | Linoleic acid | 0.021 |
| L-Glutamic | 549.65 | Phenol Red·Na | 6.56 |
| Glycine | 19.375 | Putrescine·2HCl | 0.04025 |
| L-Histidine·HCl· | 23.165 | Pyruvic acid·Na | 110 |
| L-Hydroxyproline | 5 | | |
| L-Isoleucine | 65.935 | D-Glucose | 2000 |
| L-Leucine | 68.225 | | |
| L-Lysine·HCl | 92.175 | | |
| L-Methionine | 19.87 | | |
| L-Phenylalanine | 37.99 | | |
| L-Proline | 13.625 | | |
| L-Serine | 31.125 | | |
| L·Threonine | 55.525 | | |
| L-Tryptophan | 9.76 | | |
| L-Tyrosine· | 42.36 | | |
| L-Valine | 54.825 | | |

7. A kit for culturing a human pluripotent stem cell while maintaining an undifferentiated state, comprising an activin-containing supplement, an activin-free supplement and an ingredient of basal medium for human pluripotent stem cell.

8. The kit according to claim 7, wherein the supplements comprise fibronectin, insulin, transferrin, 2-mercaptoethanol, 2-ethanolamine, sodium selenite and an albumin-conjugated oleic acid.

9. The kit according to claim 7 or 8, wherein fibronectin is added to the supplements.

10. The kit according to any one of claims 7 to 9, wherein the supplements further comprise a protein kinase C inhibitor.

11. The kit according to any one of claims 7 to 10, wherein the basal medium is a chemically defined medium comprising one or more sugars, one or more inorganic salts, one or more amino acids, one or more vitamins and a trace ingredient.

12. The kit according to any one of claims 7 to 10, wherein the basal medium is a hESF-grow medium shown in Table 2:
**[Table 2]**
| hESF-grow medium | | | |
|---|---|---|---|
| INORGANIC SALTS | mg/L | VITAMINS | mg/L |
| CaCl₂ | 108.305 | Ascorbic acid 2-phosphate | 100 |
| Ca(NO₃)₂ | 25 | D-Biotin | 0.05185 |
| CuSO₄·5H₂O | 0.000625 | Choline Chloride | 6.24 |
| Fe(NO₃)₃·9H₂O | 0.05 | Folic Acid | 2.575 |
| FeSO₄·7H₂O | 0.2085 | I-Inositol | 16.85 |
| MgCl·6H₂O | 30.515 | Niacinamide | 2.25925 |
| MgSO₄ | 61.055 | D-Pantothenic Acid·½Ca | 2.1825 |
| KCl | 355.9 | Pyridoxal·HCl | 2 |
| NaCl | 6599.75 | Pyridoxine-HCl | 0.2655 |
| Na₂HPO₄ 2H₂O | 295.28 | Riboflavin | 0.2595 |
| NaH₂O₄ H₂O | 61.51 | Thiamine·HCl | 2.335 |
| ZnSO₄·7H₂O | 0.216 | Vitamin B-12 | 0.34125 |
| AMINO ACIDS | mg/L | p-aminobenzoic acid | 0.25 |
| L-Alanine | 2.225 | | |
| L-Arginine | 50 | MISC. | mg/L |
| L-Arginine-HCl | 94.75 | NaHCO₃ | 2000 |
| L-Asparagine·H₂O | 16.2525 | Gluthathione | 0.25 |
| L-Aspartic Acid | 8.325 | Thymidine | 0.1825 |
| L-Cystine·HCl·H₂O | 47.5725 | Hypoxanthine | 1.02 |
| L-Cysteine-2HCl | 7.88 | Lipoic acid | 0.0525 |
| L-Glutamic Acid | 8.675 | Linoleic acid | 0.021 |
| L-Glutamine | 549.65 | Phenol Red·Na | 6.56 |
| Glycine | 19.375 | Putrescine·2HCl | 0.04025 |
| L-Histidine·HCl· | 23.165 | Pyruvic acid·Na | 110 |
| L-Hydroxyproline | 5 | | |
| L-Isoleucine | 65.935 | D-Glucose | 2000 |
| L-Leucine | 68.225 | | |
| L-Lysine·HCl | 92.175 | | |
| L-Methionine | 19.87 | | |
| L-Phenylalanine | 37.99 | | |
| L-Proline | 13.625 | | |
| L-Serine | 31.125 | | |
| L-Threonine | 55.525 | | |
| L Tryptophan | 9.76 | | |
| L-Tyrosine· | 42.36 | | |
| L-Valine | 54.826 | | |

13. The kit according to any one of claims 7 to 12, further comprising an antibody, a probe or a primer against an undifferentiation and/or differentiation marker.
